(19) Europäisches Patentamt
European Patent Office
Office européen des brevets

(11) EP 1 864 578 A1

(12) # EUROPEAN PATENT APPLICATION

(43) Date of publication:
**12.12.2007 Bulletin 2007/50**

(21) Application number: **07011326.1**

(22) Date of filing: **08.06.2007**

(51) Int Cl.:
***A23D 7/00*** *(2006.01)* ***A23D 7/01*** *(2006.01)*
***A23K 1/16*** *(2006.01)* ***A23L 1/275*** *(2006.01)*
***A23L 1/30*** *(2006.01)* ***A61K 9/107*** *(2006.01)*
***A61Q 1/06*** *(2006.01)*

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: **09.06.2006 JP 2006160809**
**01.06.2007 JP 2007146616**

(71) Applicant: **FUJIFILM Corporation**
**Minato-ku**
**Tokyo (JP)**

(72) Inventors:
- **Ogawa, Manabu**
**Ashigarakami-gun**
**Kanagawa (JP)**
- **Suzuki, Keiichi**
**Ashigarakami-gun**
**Kanagawa (JP)**

(74) Representative: **HOFFMANN EITLE**
**Patent- und Rechtsanwälte**
**Arabellastrasse 4**
**81925 München (DE)**

(54) **Carotenoid-containing emulsion composition, process for its production, and food and cosmetic product containing the same**

(57) A carotenoid-containing emulsion composition comprises carotenoid, wherein the composition further comprises: at least one water-soluble emulsifier in an aqueous phase; and tocopherol and lecithin in an oil phase.

EP 1 864 578 A1

## Description

## Background of the Invention

1. Field of the Invention

The present invention relates to a carotenoid-containing emulsion composition, a process for is production, and a food and a cosmetic product containing the same, and more particularly, to a carotenoid-containing emulsion composition wherein the particle size of emulsified particles is small and which has excellent storage stability, a process for its production and a food and a cosmetic product containing the same.

2. Description of the Related Art

Carotenoids are yellow to red terpenoid natural dyes which can be found in plants, algae and bacteria. Examples of the carotenoids include hydrocarbons (carotenes) and the oxidized alcohol derivatives thereof (xanthophylls). Specific examples thereof include actinioerythrol, astaxanthin, bixin, canthaxanthin, capsanthin, capsorbin, β-8'-apo-carotenal (apocarotenal), β-12'-apo'-carotenal, α-carotene, β-carotene, "carotene" (mixtures of α- and β-carotenes), γ-carotene, β-criptoxanthin, lutein, lycopene, violaxanthin, zeaxanthin and esters of them which have a hydroxyl or carboxyl group. Many of carotenoids exist in nature in the form of cis- and trans-isomers, but synthetic products are often racemic mixtures. Carotenes are generally extracted from plant materials.

For example, lutein extracted from petals of marigold is widely used as a material for feed of poultry to give its color to the skin and fats of poultry and eggs they lay. Many of carotenes are also produced by synthesis. Many of commercially available β-carotenes are produced by synthesis.

Astaxanthins (including astaxanthin and esters thereof) are widely distributed in the natural worlds of plants and animals and are mainly used as color enhancers for hatchery fish or hatchery fowls. Also, astaxantin is known to have antioxidant effect, anti-inflammatory effect (JP-A-2-49091), anti-aging effect for skin (JP-A-5-155736) and whitening effect (Nippon Koshohin Kagakukai 19th Gakujutu-taikai Koen-yosi-shu, p.66, 1994). Because of these effects, addition of astaxanthins to raw materials for foods, cosmetics and medicines and to processed products thereof have conventionally been examined and practiced.

As has been described hereinbefore, astaxanthins are widely distributed in the natural worlds of plants and animals and are obtained as natural extracts from them. In addition, since their chemical structures have already been known, they can also be obtained by organic chemical synthesis.

In Japan, however, use of them in foods, cosmetics and medicines, particularly foods or medicines for oral administration, is under legal regulations relating to natural extracts.

Natural products containing astaxanthins include hematococcus algae and euphausiids.

Also, in order to add the carotenoids as mentioned above to foods, cosmetics, medicines and other processed products, they are added as an emulsion having high dispersibility.

However, carotenoids derived from natural products have an unstable structure, and in addition, it has been difficult to stabilize the dispersed state at a high level for a comparatively long period of time with the particle size of the emulsified particles being within a satisfactory range.

Stability of dyes including carotenoids in raw materials for foods, cosmetics and medicines and processed products thereof has conventionally been an industrially important matter. Dyes in foods, cosmetics and medicines are generally decomposed by causes such as ultraviolet rays, oxygen, enzymes, heat, moisture and light. As methods for stabilizing dyes, there have so far been made various techniques.

In particular, JP-A-9-328419and JP-T-2005-506841 (the term "JP-T" as used herein means a published Japanese translation of a PCT patent application) describe the technique regarding dispersion stability of carotenoid series dyes.

## Summary of the Invention

In the case of preparing a carotenoid-containing emulsion composition, however, it has been difficult for even the techniques described in JP-A-9-328419 and JP-T-2005-506841 to stabilize carotenoids at a high level for a comparatively long period of time.

An object of the invention is to provide a carotenoid-containing emulsion composition which overcomes the defects with the above-mentioned techniques, wherein the particle size of emulsified particles is small, and which has excellent storage stability, a process for its production and a food and a cosmetic product containing the same.

As a result of intensive investigations, the inventors have found that the defects with the above-mentioned conventional techniques can be overcome by employing the following constitution.;

That is, the invention is as follows.

(1) A carotenoid-containing emulsion composition, which comprises carotenoid, wherein the composition further comprises:

at least one water-soluble emulsifier in an aqueous phase; and
tocopherol and lecithin in an oil phase.

(2) The carotenoid-containing emulsion composition as described in (1) above, wherein the carotenoid is at least one of astaxanthin and an ester thereof.
(3) The carotenoid-containing emulsion composition as described in (1) or (2) above, wherein the at least one water-soluble emulsifier has an HLB of 10 or more.
(4) The carotenoid-containing emulsion composition as described in any of (1) to (3) above, wherein the at least one water-soluble emulsifier is selected from the group consisting of sucrose fatty acid esters, polyglycerin fatty acid esters and sorbitan fatty acid esters.
(5) The carotenoid-containing emulsion composition as described in any of (1) to (4) above, wherein a content of the carotenoid is from 0.1 to 10% by mass based on a mass of the composition, a content of the lecithin is from 0.1 to 10% by mass based on a mass of the composition, and a content of the tocopherol is from 0 . 1 to 5% by mass based on a mass of the carotenoid.
(6) The carotenoid-containing emulsion composition as described in any of (1) to (5) above, which further comprises glycerin.
(7) The carotenoid-containing emulsion composition as described in any of (1) to (6) above, wherein a content of the glycerin is from 10 to 60% by mass based on a mass of the composition.
(8) The carotenoid-containing emulsion composition as described in any of (1) to (7) above, which further comprises an antioxidant.
(9) The carotenoid-containing emulsion composition as described in (8) above, wherein the antioxidant is a radical scavenger.
(10) The carotenoid-containing emulsion composition as described in any of (1) to (9) above, wherein a particle size of emulsified particles is 200 nm or less.
(11) A process for producing the carotenoid-containing emulsion composition as described in any of (1) to (10) above, which comprises the following steps of:

a) dissolving the at least one water-soluble emulsifier in an aqueous medium to obtain an aqueous phase;
b) mixing and dissolving the carotenoid, the tocopherol and the lecithin, and optionally other fats and oils to obtain an oil phase; and
c) mixing the aqueous phase with the oil phase under stirring to obtain the emulsion composition.

(12) The process as described in (11) above, which further comprises:

performing high-pressure emulsification to reduce a particle size of emulsified particles of the emulsion composition.

(13) A food, which comprises the carotenoid-containing emulsion composition as described in any of (1) to (10) above.
(14) A cosmetic product, which comprises the carotenoid-containing emulsion composition as described in any of (1) to (10) above.

**Detailed Description of the Invention**

The carotenoid-containing emulsion composition of the invention will be described in detail below.
The carotenoid-containing emulsion composition of the invention is characterized in that it contains at least one water-soluble emulsifier in the aqueous phase and contains tocopherol and lecithin in the oil phase.
The carotenoids which the emulsion composition of the invention contains are as described in the item of [Description of the Related Art] in this specification, and preferred examples thereof include actinioerythrol, astaxanthin, bixin, canthaxanthin, capsanthin, β-8'-apo-carotenal (apocarotenal), β-12'-apo'-carotenal, α-carotene, β-carotene, "carotene" (mixtures of α- and β-carotenes), γ-carotene, β-criptoxanthin, lutein (xanthophyll), lycopene, violaxanthin, zeaxanthin and esters of them which have a hydroxyl or carboxyl group.
Of these, addition of astaxanthin to raw materials for foods, cosmetics and medicines and to processed products thereof has conventionally been demanded, examined and practiced because it has antioxidant effect, anti-inflammatory

effect, anti-aging effect for skin and whitening effect.

Astaxanthin is a red dye having an absorption maximum at 476 nm (ethanol) and 468 nm (hexane) and belongs to xanthophylls, one kind of carotenoids (Davies, B.H.; Chemistry and Biochemistry of Plant Pigments, T. W. Goodwin ed., 2nd ed. 38-165, Academic Press, NY, 1976). The chemical structure of astaxanthin is 3,3'-dihydroxy-β,β-carotene-4,4'-dione ($C_{40}H_{52}O_4$; molecular mass: 596.82) and its chemical formula is represented by the following formula (1).

Formula (1)

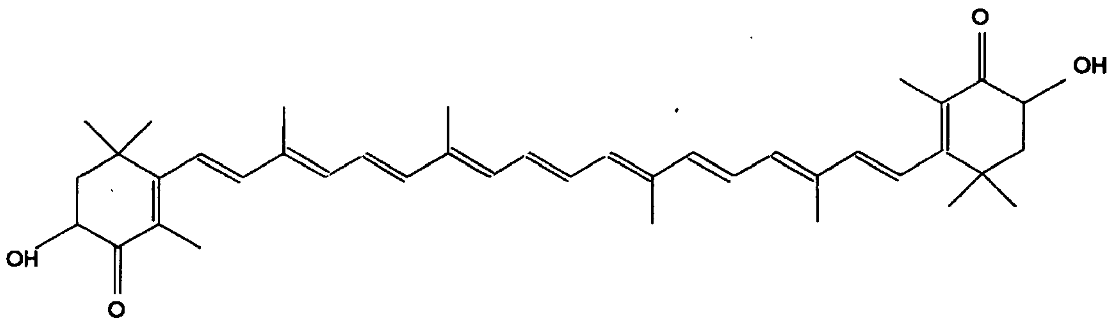

Astaxanthin and esters thereof were first isolated from lobster (Astacus gammarus L.) and their assumed structures were disclosed by R. Kuhn et al. (Kuhn, R. , Soerensen, N.A. : The coloring matters of the lobster (Astacus gammarus L.), Z. Angew. Chem., 1938, 51, pp.465-466). Since then, it has been made clear that astaxanthin is widely distributed in the natural world and usually exists as fatty acid esters thereof and that it exists in Crustacea also as an astaxanthin protein (ovorubin, crustacyanine) wherein astaxanthin is bound to a protein (Cheesman, D.F.: Ovorubin, a chromoprotein from the eggs of the gastropod mollusc Pomacea canaliculata, Proc. Roy. Soc. B, 1958, 149, pp.571-587).

Isomers of astaxanthin exist which are different from each other in configuration of the hydroxyl groups at 3- and 3' -positions of the ring structures on both ends of the molecule. They are three isomers of 3S,3S'-isomer, 3S,3R'-isomer (meso isomer) and 3R,3R'-isomer. Further, there exist cis- and trans-isomers for the conjugated double bonds in the central part of the molecule. They are, for example, all is-isomer, 9-cis-isomer and 13-cis isomer.

The hydroxyl groups at the 3- and 3'-positions can form esters with a fatty acid. Astaxanthin obtained from euphausiid is a diester wherein two fatty acids are respectively bound to the hydroxyl groups of astaxanthin (Yamaguchi, K., Miki, W., Toriu, N., Kondo, Y., Murakami, M., Konosu, S., Satake, M. and Fujita, T. : The composition of carotenoid pigments in the Antarctic krill Euphausia superba, Bull. Jap. Soc. Sci. Fish., 1983, 49, pp.1411-1415), and astaxanthin obtained from H. pulvialis is a 3S, 3S'-isomer most of which is in the form of being mono-esterified with a fatty acid (Renstrom, B. and Liaaen-Jensen, S. : Fatty acids of some esterified carotenols, Comp. Biochem. Physiol. B., Comp. Biochem., 1981, 69, pp. 625-627) . Also, astaxanthin obtained from Phaffia Rhodozyma is a 3R,3R'-isomer (Andrewes, A.G. and Starr, M.P.: (3R,3'R)-Asttaxanthin from the yeast Phaffa rhodozyma, Phytochem., 1976, 15, pp.1009-1011) which has an opposite structure of the 3S,3S'-isomer usually found in nature and is in a free form of not forming an ester with a fatty acid ( Andrewes, A.G., Phaffia, H.J. and Starr, M. P. : Carotenids of Phaffa rhodozyma, a red pigmented fermenting yeast, Phytochem., 1976, 15, pp.1003-1007).

Extracts of natural products containing astaxanthin and the ester thereof to be used in the invention are not particularly limited and are exemplified by an extract of Haematococcus alga and an extract of euphausiid, with the extract of Haematococcus alga being common.

An extract of Haematococcus alga (Haematococcus algae-derived dye) is known to be different from the dye derived from euphausiid and synthesized astaxanthin.

Specific examples of the origin of an extract of Haematococcus alga include Haematococcus pluvialis, Haematococcus lacustris, Haematococcus capensis, Haematococcus droebakensis and Haematococcus zimbabwiensis.

Methods for culturing an alga Haematococcus employable in the invention are not particularly limited and various methods disclosed in JP-A-8-103288, etc. can be employed. It suffices to change the morphology of the alga cells from vegetative cells to resting cells of cyst cells.

An extract of Haematococcus alga to be used in the invention can be obtained by, as needed, crushing cell wall by a method described in, for example, JP-A-5-68585, adding to the crushed product an extracting solvent such as an organic solvent of acetone, ether, chloroform or alcohol (e.g., ethanol or methanol) or ultra-critical state carbon dioxide, followed by extracting. Also, products widely commercially sold can be used, and examples thereof include ASTOTS-S, ASTOTS-2.50, ASTOTS-50 and ASTOTS-100 manufactured by Takedashiki Co., Ltd., AstaREAL oil 50F and AstaREAL 5F manufactured by Fuhi Chemical Industy Co., Ltd. and BioAstin SCE7 manufactured by Toyo Koso Kagaku

Co., Ltd.

The content of the pure dye component in the extract of Haematococcus alga to be used in the invention is preferably from 0.001 to 50% by mass, more preferably from 0.01 to 25% by mass. (In this specification, mass ratio is equal to weight ratio.)

Additionally, the extract of Haematococcus to be used in the invention contains astaxanthin or its ester derivative as a pure dye component as is the same with the dyes described in JP-A-2-49091, with the content of the ester derivative being generally 50% or more, preferably 75% or more, more preferably 90% or more. More detailed descriptions are given in Astaxanthin no Kagaku (Chemistry of Astaxanthin), 2005, Internet <URL:http://www.astaxanthin.co.jp/chemical/basic.htm>

The content of a carotenoid in the carotenoid-containing emulsion composition of the invention is preferably from 0.1 to 10% by mass, more preferably from 0.5 to 5% by mass, more preferably from 0.2 to 2% by mass.

Next, emulsifiers to be used in the carotenoid-containing emulsion composition of the invention will be described below.

The water-soluble emulsifiers which can be used in the invention are not particularly limited as long as they are emulsifiers soluble in an aqueous medium. For example, nonionic surfactants having an HLB of 10 or more, preferably 12 or more, are preferred. With emulsifiers having a too low HLB, there might result insufficient emulsifying force.

Here, HLB means a hydrophile-lipophile balance which is commonly used in the field of surfactants, and can be calculated according to a commonly employed calculating formula, for example, Kawakami formula. Kawakami formula is shown below:

$$HLB = 7 + 11.7\log(Mw/Mo)$$

wherein Mw represents the molecular mass of the hydrophilic group, and Mo represents the molecular mass of the oleophilic group.

It is also possible to use numerical values described in catalogues or the like as HLB values.

As can be seen from the above formula, too, it is possible to obtain an emulsifier having any HLB value by utilizing additivity relationship of HLB values.

Emulsifiers which can be used in the invention are not particularly limited, with nonionic surfactants being preferred. Examples of the nonionic surfactants include glycerin fatty acid esters, organic acid mono glycerides, polyglycerin fatty acid esters, propylene glycol fatty acid esters, polyglycerin condensed ricinoleic acid ester, sorbitan fatty acid esters and sucrose fatty acid esters. Polyglycerin fatty acid esters, sorbitan fatty acid esters and sucrose fatty acid esters are more preferred. The above-mentioned emulsifiers are not necessarily products highly purified by distillation or the like, and may be reaction mixtures.

The polyglycerin fatty acid esters to be used in the invention are esters between a polyglycerin having an average polymerization degree of 2 or more, preferably from 6 to 15, more preferably from 8 to 10 and a fatty acid containing from 8 to 18 carbon atoms such as caprylic acid, capric acid, lauric acid, myristic acid, palmitic acid, stearic acid, oleic acid or linoleic acid. Preferred examples of the polyglycerin fatty acid esters include hexaglycerin monooleate, hexaglycerin monostearate, hexaglycerin monopalmitate, hexaglycerin monomyristate, hexaglycerin monolaurate, decaglycerin monooleate, decaglycerin monostearate, decaglycerin monopalmitate, decaglycerin monomyristate and decaglycerin monolaurate. These polyglycerin fatty acid esters can be used independently or as a mixture thereof. Examples of commercially available products include NIKKOL DGMS, NIKKOL DGMO-CV, NIKKOL DGMO-90V, NIKKOL DGDO, NIKKOL DGMIS, NIKKOL DGTIS, NIKKOL Tetraglyn 1-SV, NIKKOL Tetraglyn 1-O, NIKKOL Tetraglyn 3-S, NIKKOL Tetraglyn 5-S, NIKKOL Tetraglyn 5-O, NIKKOL Hexaglyn 1-L, NIKKOL Hexaglyn 1-M, NIKKOL Hexaglyn 1-SV, NIKKOL Hexaglyn 1-O, NIKKOL Hexaglyn 3-S, NIKKOL Hexaglyn 4-B, NIKKOL Hexaglyn 5-S, NIKKOL Hexaglyn 5-O, NIKKOL Hexaglyn PR-15, NIKKOL Decaglyn 1-L, NIKKOL Decaglyn 1-M, NIKKOL Decaglyn 1-SV, NIKKOL Decaglyn 1-50SV, NIKKOL Decaglyn 1-ISV, NIKKOL Decaglyn 1-O, NIKKOL Decaglyn 1-OV, NIKKOL Decaglyn 1-LN, NIKKOL Decaglyn 2-SV, NIKKOL Decaglyn 2-ISV, NIKKOL Decaglyn 3-SV, NIKKOL Decaglyn 3-OV, NIKKOL Decaglyn 5-SV, NIKKOL Decaglyn 5-HS, NIKKOL Decaglyn 5-IS, NIKKOL Decaglyn 5-OV, NIKKOL Decaglyn 5-O-R, NIKKOL Decaglyn 7-S, NIKKOL Decaglyn 7-O, NIKKOL Decaglyn 10-SV, NIKKOL Decaglyn 10-IS, NIKKOL Decaglyn 10-OV, NIKKOL Decaglyn 10-MAC and NIKKOL Decaglyn PR-20 manufactured by Nikko Chemicals Co., Ltd., Ryoto-Polyglyester L-10D, L-7D, M-10D, M7D, P-8D, S-28D, S-24D, SWA-20D, SWA-15D, SWA-10D, O-50D, O-15D, B-100D, B-70D and ER-60D manufactured by Mitsubishi Chemical Foods Co., Ltd., Sunsoft Q-17UL, Sunsoft Q-14S and Sunsoft A-141C manufactured by Taiyo Kagaku Co., Ltd. and Poem DO-100 and Poem J-0021 manufactured by Riken Vitamin Co., Ltd.

The sorbitan fatty acid esters to be used in the invention contain preferably 8 or more carbon atoms in the fatty acid moiety, more preferably 12 or more carbon atoms. Preferred examples of the sorbitan fatty acid esters include

sorbitan monocaprylate, sorbitan monolaurate, sorbitan monostearate, sorbitan sesquistearate, sorbitan tristearate, sorbitan isostearate, sorbitan sesquiisostearate, sorbitan oleate, sorbitan sesquioleate and sorbitan trioleate. These sorbitan fatty acid esters can be used independently or as a mixture thereof. Examples of commercially available products include NIKKOL SL-10, SP-10V, SS-10V, SS-10MV, SS-15V, SS-30V, SI-10RV, SI-15RV, SO-10V, SO-15MV, SO-15V, SO-30V, SO-10R, SO-15R, SO-30R and SO-15EX manufactured by Nikko Chemicals Co., Ltd. and Solgen 30V, 40V, 50V, 90 and 110 manufactured by Dai-ichi Kogyo Seiyaku Co., Ltd.

The sucrose fatty acid esters to be used in the invention contains preferably 12 or more carbon atoms in the fatty acid moiety, more preferably from 12 to 20 carbon atoms. Preferred examples of the sucrose fatty acid esters include sucrose dioleate, sucrose distearate, sucrose dipalmitate, sucrose dimyristate, sucrose dilaurate, sucrose monooleate, sucrose monostearate, sucrose monopalmitate, sucrose monomyristate and sucrose monolaurate. In the invention, these sucrose fatty acid esters can be used independently or as a mixture. Examples of commercially available products include Ryoto Sugar Ester S-070, S-170, S-270, S-370, S-370F, S-570, S-770, S-970, S-1170, S-1170F, S-1570, S-1670, P-070, P-170, P-1570, P-1670, M-1695, O-170, O-1570, OWA-1570, L-195, L-595, L-1695, LWA-1570, B-370, B-370F, ER-190, ER-290 and POS-135 manufactured by Mitsubishi Chemical Foods Co., Ltd. and DK ester SS, F160, F140, F110, F90, F70, F50, F-A50, F-20W, F-10, F-A10E, Cosmelike B-30, S-10, S-50, S-70, S-110, S-160, S-190, SA-10, SA-50, P-10, P-160, M-160, L-10, L-50, L-160, L-150A, L-160A, R-10, R-20, 0-10 and 0-150 manufactured by Dai-ichi Kogyo Seiyaku Co., Ltd.

The addition amount of these emulsifiers is preferably from 0.1 to 50% by mass, more preferably from 0.5 to 20% by mass, still more preferably from 1 to 15% by mass, based on the mass of the emulsion composition.

In case when the addition amount is too small, there arise problems that an emulsion containing emulsified particles having a fine particle size cannot be obtained and that stability of the resulting emulsion is insufficient.

On the other hand, in case when the addition amount is too large, there arises a problem that foaming of the emulsion becomes serious.

Tocopherol to be used in the carotenoid-containing emulsion composition of the invention is not particularly limited, and is selected from a group of compounds consisting of tocopherol and the derivatives thereof.

The group of compounds consisting of tocopherol and the derivatives thereof include tocopherol and the derivatives thereof such as dl-α-tocopherol, d1-β-tocopherol, dl-γ-tocopherol, dl-δ-tocopherol, dl-α-tocopherol acetate, dl-α-tocopherol nicotinate, d1-α-tocopherol linoleate and dl-α-tocopherol succinate; α-tocotrienol; β-tocotrienol; γ-tocotrienol; and δ-tocotrienol. These are often used in a state of a mixture thereof, and can be used in a state called extracted tocopherol or mix tocopherol.

In the carotenoid-containing emulsion composition of the invention, the content of tocopherol is not particularly limited, but is preferably in a ratio of from 0.1 to 5, more preferably from 0.2 to 3, still more preferably from 0.5 to 2, based on the mass of carotenoids.

Lecithin to be used in the invention is a compound which contains a glycerin skeleton, fatty acid residues and a phosphoric acid residue as necessary constituents and wherein a base or a polyhydric alcohol is bound to them. It is also called a phospholipid. Since lecithin has both a hydrophilic group and a hydrophobic group within the molecule, it has conventionally been used as an emulsifier in the field of foods, medicines and cosmetics.

It has been known that anti-oxidant effect of oils and fats can be improved by combining an antioxidant with lecithin. In the invention, it has been found that the combination of lecithin and tocopherol is effective for preventing oxidation of natural dyes such as carotenoids as well as oxidation of oils and fats.

Industrially, a lecithin product having a lecithin purity of 60% or more is utilized as lecithin and can be utilized in the invention. Preferably, however, a lecithin product generally called high-purity lecithin is used in the invention, which has a lecithin purity of 80% or more, more preferably 90% or more. This lecithin purity can be determined by subtracting the mass of a toluene-insoluble component and the mass of an acetone-soluble component utilizing lecithin's properties that it is readily soluble in toluene and insoluble in acetone.

As lecithin, there can be illustrated conventionally known various products separated by extraction from organisms of plants, animals and microbes. Specific examples of such lecithin include various lecithins derived from plants such as soybeans, corn, peanuts, rape seeds and wheat; animals such as egg yolk and cow; and microbes such as Escherichia coli. To give chemical names of these lecithins, there can be illustrated glycerolecithins such as phosphatidic acid, phosphatidyl glycerin, phosphatidyl inositol, phosphatidyl ethanolamine, phosphatidyl methylethanolamine, phosphatidyl choline, phosphatidyl serine, bis phosphatidic acid and diphosphatidyl glycerin (cardiolipin); and sphingolecithins such as sphingomyelin.

Also, in the invention, hydrogenated lecithin, enzyme-decomposed lecithin, enzyme-decomposed and hydrogenated lecithin and hydroxylecithin can be used as well as the above-mentioned high-purity lecithin. These lecithins to be used in the invention can be used independently or in the form of a mixture of plural kinds thereof.

In the carotenoid-containing emulsion composition of the invention, the content of lecithin is preferably from 0.1 to 10% by mass, more preferably from 0.2 to 5% by mass, still more preferably from 0.5 to 2% by mass, based on. the mass of the composition.

It is preferred for the carotenoid-containing emulsion composition of the invention to contain glycerin, because glycerin serves to more reduce the particle size of emulsified particles of the emulsion and stably keep the small particle size for a long period of time.

In this case, the content of glycerin is preferably from 10 to 60% by mass, more preferably from 20 to 55% by mass, still more preferably from 30 to 50% by mass, based on the mass of the composition of the invention.

It is also preferred for the carotenoid-containing emulsion composition of the invention to contain an antioxidant.

Antioxidants to be used in the carotenoid-containing emulsion composition of the invention are not particularly limited, and are exemplified by (a) a group of compounds consisting of ascorbic acid, erisorbic acid, salts thereof, ascorbic acid derivatives, erisorbic acid derivatives and salts thereof, (b) a group of compounds consisting of polyphenols and (c) radical scavengers.

Regarding the antioxidants to be used in the carotenoid-containing emulsion composition of the invention, hydrophilic antioxidants and/or oil-soluble antioxidants can be used independently or in combination thereof. For example, as the hydrophilic antioxidants, there can be illustrated compounds belonging to the compound group (a) whereas, as the oil-soluble antioxidants, there can be illustrated compounds belonging to the compound group (b).

The content of the antioxidant in the carotenoid-containing emulsion composition of the invention is generally from 0.1 to 10% by mass, preferably from 0.5 to 5% by mass, more preferably from 0.2 to 2% by mass.

Specific examples of the compound groups (a) to (c) to be used in the carotenoid-containing emulsion composition of the invention will be described below which, however, do not limit antioxidants which can be used in the invention.

(a) As ascorbic acid, erisorbic acid, salts thereof, ascorbic acid derivatives, erisorbic acid derivatives and salts thereof, there are illustrated L-ascorbic acid, sodium L-ascorbate, potassium L-ascorbate, calcium L-ascorbate, L-ascorbyl phosphate, magnesium L-ascorbyl phosphate, L-ascorbyl sulfate, disodium L-ascorbyl sulfate and L-ascorbyl 2-glucoside. Of these, L-ascorbic acid, sodium L-ascorbate, L-ascorbyl 2-glucoside, magnesium L-ascorbyl phosphate and disodium L-ascorbyl sulfate are particularly preferred.

As erisorbic acid, erisorbic acid derivatives and salts thereof, there are illustrated erisorbic acid, sodium erisorbate, potassium erisorbate, calcium erisorbate, erisorbyl phosphate and erisorbyl sulfate. Of these, erisorbic acid and sodium erisorbate are particularly preferred.

As the antioxidants belonging to the compound group (a) to be used in the invention, commercially available ones can properly be used. Examples thereof include L-ascorbic acid (Takeda Pharmaceutical Company Limited, Fuso Chemical Co., Ltd., BASF Japan, Dai-ichi Seiyaku Co., Ltd., etc.), sodium L-ascorbate (Takeda Pharmaceutical Company Limited, Fuso Chemical Co., Ltd., BASF Japan, Dai-ichi Seiyaku Co., Ltd., etc.), ascorbyl 2-glucoside (trade name: AA-2G; manufactured by K.K. Hayashibara Seibutsu Kagaku Kenkyujo), and magnesium L-ascorbyl phosphate (trade name: Ascorbic acid PM "SDK"; manufactured by Showa Denko K. K. ; trade name: NIKKOL VC-PMG; manufactured by Nikko Chemicals Co., Ltd.; trade name: C-MATE; manufactured by Takeda Pharmaceutical Company Limited).

(b) Compound group consisting of polyphenols

As compounds of the compound group consisting of polyphenols, there can be illustrated flavonoids (e.g., catechin, anthocyanin, flavon, isoflavon, flavan, flavanone and rutin), phenolic acids (e.g., chlorogenic acid, ellagic acid, gallic acid and propyl gallate), lignans, curcumins and coumarins. Since these compounds are contained in large quantities in extracts obtained from natural products as follows, they can be utilized in the form of extracts.

Examples thereof include licorice extract (extract of Glycyrrhizae Radix), cucumber extract, keiketto extract (extract of dried stem of Leguminosae, Millettia reticulate Benth. and Mucuna birdwoodiana Tutcher), Gentiana Lutea (gentian) extract, Geranium Thunbergii extract, cholesterol and the derivatives thereof, Crataegus Cuneata extract, Paeoniae Radix extract, ginkgo extract, Scutellaria baicalensis Georgi (Scutellariae Radix) extract, carrot extract, Rosa rugosa (Japanese rose) extract, Sanpenzu (Cassia nomame) extract, Potentilla tormentilla extract, parsley extract, peoney (Moutan Cortex) extract, Mokka (Chaenomeles lagenariakoidz.) extract, Melissa Officiaalis extract, Yasha-jitsu (cornflower) extract, Saxifraga Sarmentosa extract, Rosmarinus officinalis (rosemary) extract, lettuce extract, tea extract (oolong tea, black tea, green tea, etc.), fermentation products of microorganisms and Fructus Momordicae extract. (Terms in the parentheses are other names of plants, names of galenicals, and the like). Of these polyphenols, catechin, rosemary extract, glucosyl rutin, ellagic acid and gallic acid are particularly preferred.

As the antioxidants belonging to the compound group (b) to be used in the invention, commercially available products can generally be used. For example, there can be illustrated ellagic acid (Wako Pure Chemical Industries, Ltd.; etc.), rosemary extract (trade name: RM-21A, RM-21E; Mitsubishi Chemical Foods Co., Ltd.; etc.), catechin (trade name: Sankatol w-5, No. 1; manufactured by Taiyo Kagaku Co., Ltd.; etc.) and rutin•glucosyl rutin•enzyme-decomposed rutin (trade name: rutin K-2, P-10; Kiriya Chemical Co, Ltd.; trade name: αG Rutin: Hayashibara Seibutsu Kagaku Kenkyujo; etc.).

(c) Group consisting of radical scavengers

A radical scavenger is an additive which plays a role of suppressing generation of radicals and scavenging generated radicals as rapidly as possible to thereby interrupt chain reaction (source: Yukagaku Binran 4th ed. compiled by Nihon Yukagakukai, 2001). As a direct method for confirming the function as radical scavenger, there are known methods of measuring the state of scavenging radicals by means of a spectrophotometer or ESR (an electron spin resonance apparatus). In these methods, DPPH (1,1-diphenyl-2-picrylhydrazyl) or garbinoxyl radical is used as a reagent.

In the invention, the radical scavenger is a compound which prolongs the time required for raising the peroxide value (POV value) of an oil to 60 meq/kg two times or more, more preferably 5 times or more, in comparison with a blank test under the following experimental conditions utilizing the auto-oxidation reaction of fats and oils.
Oil: olive oil
Addition amount: 0.1% by mass based on the mass of the oil
Testing conditions: A sample was heated at 190°C, the POV value was measured with time, and the time at which the POV becomes 60 meq/kg was calculated.
Compounds to be used in the invention as radical scavengers, any of various antioxidants which are described in Kosankazai no Riron to Jissai (Theory and Practice of Antioxidants) written by Kajimoto and published by San Shobo in year 1984 and Sanka Bosizai Handobukku (Handbook of Antioxidants) written by Sawatari, Nishino and Tabata and published by Taiseisha in year 1976 and which function as radical scavengers can be used. Specifrically, there are illustrated compounds having phenolic OH, amine series antioxidants such as phenylenediamine and oil-soluble derivatives of ascorbic acid and erythorbic acid.
Preferred compounds are illustrated below which, however, do not limit the invention in any way.
Examples of the compounds having phenolic OH group include guaiacum resin, nordihydroguaiaretic acid (NDGA), gallic acid esters, BHT (butylhydroxytoluene), BHA (butylhydroxyanisole), tocopherols and bisphenols. Examples of the gallic acid esters include propyl gallate, butyl gallate and octyl gallate.
Examples of the amine series compounds include phenylenedieminas, with diphenyl-p-phenylenediamine or 4-amino-p-diphenylamine being more preferred.
Examples of the oil-soluble derivatives of ascorbic acid and erythorbic acid include L-ascorbyl stearate, L-ascorbyl tetraisopalmitate, L-ascorobyl palmitate, erythorbyl palmitate and erythorbyl tetraisopalmitate.
The particle size of emulsified particles of the carotenoid-containing emulsion of the invention is not particularly limited, but is preferably 200 nm or less, more preferably from 5 to 100 nm.
Although the particle size varies depending upon factors such as stirring conditions (shearing force, temperature and pressure), amounts of additives used, ratio of the oil phase to the aqueous phase, and amounts of surfactants used, there arises no practical problem as long as the particle size is within the range of the invention. The emulsion composition of the invention can be subjected to measurement by means of a particle size distribution-measuring apparatus.

<Process for producing the emulsion composition>

Processes for producing the carotenoid-containing emulsion composition of the invention are not particularly limited, but a process to obtain an emulsion composition which comprises, for example, the steps of a) dissolving a water-soluble emulsifier in an aqueous medium to obtain an aqueous phase, b) mixing carotenoid, tocopherol, lecithin, and as needed, other oils and fats to dissolve and obtain an oil phase, and c) mixing the aqueous phase and the oil phase under stirring to emulsify and disperse is preferred.
Upon emulsification and dispersion, it is particularly preferred to employ two or more emulsifying apparatuses by, for example, first emulsifying a normal emulsifying machine utilizing shearing force such as a stirrer, an impeller, a homomixer or a continuous flow shear apparatus, then passing through a high-pressure homogenizer. Employment of the high-pressure homogenizer serves to make the particle size of finely emulsified oil droplets of the resulting emulsion more uniform. It is also possible to conduct the emulsifying procedure plural times for the purpose of obtaining oil droplets having a still more uniform particle size.
As the high-pressure homogenizer, there are illustrated a chamber type high-pressure homogenizer having a chamber wherein the flow passage for a solution to be processed is fixed and a homovalve type high-pressure homogenizer having a homovalve. Of these, a homovalve type high-pressure homogenizer is widely used particularly in the emulsification field such as foods or cosmetics since it permits easy adjustment of the width of a flow passage for a solution to be processed and provides a wide operation range. On the other hand, a chamber type high-pressure homogenizer is used for use which requires a super-high pressure since it facilitates construction of a pressure-increasing mechanism though degrees of freedom with respect to operation are low.
As the chamber type high-pressure homogenizer, there are illustrated Microfluidizer (manufactured by Microfluidics), Nanomizer (manufactured by Yoshida Kikai Co., Ltd.) and Altimizer (manufactured by Sugino Machine K.K.).
As the homovalve type high-pressure homogenizer, there are illustrated Gaulin type homogenizer (manufactured by APV), Rannie type homogenizer (manufactured by Rannie), high-pressure homogenizer (manufactured by Niro Soavi), homogenizer (Sanwa Machine Co., Inc.), high-pressure homogenizer (manufactured by Izumi Food Machinery Co., Ltd.) and ultra-high-pressure homogenizer (manufactured by IKA).

Dispersion by means of a high-pressure homogenizer is considered to be conducted by a large shear force generated when a liquid passes through an extremely narrow (small) gap at a high speed. This shear force is almost proportional to the pressure, and a higher pressure generates a stronger shear force, i.e., a dispersing force to be applied to particles dispersed in the liquid. However, most of kinetic energy generated when a liquid flows at a high speed is converted to heat, and hence a higher pressure more increases the temperature of the liquid, which might result in deterioration of dispersion components or acceleration of re-agglomeration of emulsified particles. Therefore, there exists the optimal level with respect to pressure of the high-pressure homogenizer. This optimal level is considered to vary dependent upon materials to be dispersed and targeted particle size. In the invention, the pressure of the homogenizer for the processing is preferably 50 MPa or more, more preferably from 50 to 250 MPa, still more preferably from 100 to 250 MPa. Also, it is preferred to cool the emulsion by passing it through some cooling apparatus within 30 seconds, preferably within 3 seconds, from immediately after passing through the chamber.

As another effective process for obtaining a fine emulsion, there can be illustrated use of an ultrasonic homogenizer. Specifically, there has been known a method of emulsifying using the common emulsifying apparatus as described above utilizing the shearing action, and then irradiating with ultrasonic waves of from 15 to 40 kHz in frequency. However, there have been no commercially available ultrasonic wave-generating apparatuses which can irradiate with enough scale and, with small apparatuses, there has been a limit as to processible volume of a liquid medium. Therefore, although the process of producing an emulsion using such ultrasonic wave-generating apparatus is excellent in view of properties of the resulting emulsion, the processible volume is so small that it has been difficult to produce the emulsion by the process on an industrial scale.

Recently, ultrasonic wave-irradiating apparatuses have increasingly acquired high output, and mass production on a certain scale has become possible. Examples of such high output homogenizer include Ultrasonic homogenizer US-1200T, Ultrasonic homogenizer RUS-1200T and Ultrasonic homogenizer MUS-1200T (these being manufactured by Nihonseiki Kaisha Ltd.), Ultrasonic Processor UIP2000, Ultrasonic Processor UIP-4000, Ultrasonic Processor UIP-8000 and Ultrasonic Processor UIP-16000 (these being manufactured by Hielscher). These high output ultrasonic wave-irradiating apparatuses have enabled one to attain fine emulsification with a frequency of 25 kHz or less, preferably from 15 to 20 kHz, and an energy density in the dispersing area of 100 $W/cm^2$ or more, preferably 120 $W/cm^2$.

Irradiation with ultrasonic waves may be conducted batch-wise. In this case, means for stirring the whole dispersion is preferably employed in combination therewith. As such stirring means to be employed in combination, an agitator, a magnetic stirrer or a disper is employed. More preferably, flow type irradiation with ultrasonic waves can be conducted. The flow type is a type wherein a solution to be dispersed is fed at a constant flow rate to a chamber equipped with a tank for feeding a solution to be dispersed, a feeding pump and an ultrasonic wave-irradiating zone. The direction of feeding the solution to the chamber may be any direction, but a method of feeding so that the flow of the solution vertically collides with the ultrasonic wave-irradiating plane is particularly preferred.

The time of irradiating ultrasonic waves is not particularly limited, but is preferably from 2 to 200 minutes/kg in terms of time of substantially irradiating with ultrasonic waves within the vessel. Too short irradiation would result in insufficient emulsification, whereas too long irradiation might cause re-agglomeration. The optimal irradiation time varies depending upon emulsified products, and is generally preferably between 10 minutes and 100 minutes.

Since there exists the possibility that deterioration of constituents in the emulsified product or re-agglomeration of emulsified particles might occur due to increase in temperature of the emulsified solution by irradiation with ultrasonic waves having high energy density, it is preferred to employ cooling means in combination. With the batch-wise irradiation, it is possible to cool the irradiating vessel from outside or to provide a cooling unit within the vessel. With the flow type irradiation, it is preferred to provide cooling means such as a heat exchanger in the course of flow circulation as well as to cool the ultrasonic wave-irradiating chamber from outside.

A more preferred dispersion can be attained by employing the aforesaid ultra-high pressure homogenizer in combination with the ultrasonic homogenizer. That is, dispersing efficiency of the ultra-high pressure homogenizer can be enhanced by conducting dispersion in the ultra-high pressure homogenizer after emulsifying by means of a common emulsifying apparatus utilizing shearing action, which serves to reduce the number of passing the solution and enables one to obtain an emulsified product with a higher quality by reducing the number of coarse particles. In addition, the number of coarse particles can be reduced by further conducting irradiation with ultrasonic waves after conducting emulsification using the ultra-high pressure homogenizer. Further, the ultra-high pressure dispersion and the irradiation with ultrasonic waves can be repeatedly conducted in any order.

[Examples]

The invention will be described by reference to Examples which, however, do not limit the invention in any way.

The following components are dissolved under heating at 70°C for 1 hour to obtain an aqueous phase composition.

| | |
|---|---|
| Sucrose oleate (HLB=15) | 13 g |
| Decaglyceryl mono-oleate (HLB=12) | 25 g |
| Glycerin | 500 g |
| Pure water | 322 g |

Also, the following components are dissolved under heating at 70°C for 1 hour to obtain an oil phase composition.

| | |
|---|---|
| Extract of Haematococcus alga (content of astaxanthins: 20% by mass) | 40 g |
| Mix tocopherol | 10 g |
| Lecithin (from soy bean) | 90 g |

The aqueous phase is stirred in a homogenizer (10000 rpm) while keeping the temperature at 70°C, and the above-described oil phase is added thereto to obtain an emulsion. The resultant emulsion is subjected to high-pressure emulsification under a pressure of 200 MPa using Altimizer HJP-25005 (manufactured by Sugino Machine Limited).

Subsequently, the product is filtered through a microfilter of 1 μm in average pore size to prepare an astaxanthin-containing emulsion E-01.

Astaxanthin-containing compositions E-02 to 10 are prepared in the same manner as described above except for employing the formulation according to the following Table 1.

Table 1

| | | E-01 Present Invention | E-02 Present Invention | E-03 Present Invention | E-04 Present Invention | E-05 Present Invention | E-06 Present Invention |
|---|---|---|---|---|---|---|---|
| Aqueous phase | Sucrose oleate | 13 g | 13 g | 13 g | 13 g | 13 g | 13 g |
| | Decaglyceryl monooleate | 25 g | 25 g | 25 g | 25 g | 25 g | 25 g |
| | Glycerin | 500 g | 500 g | 500 g | 500 g | 500 g | 500 g |
| | Pure water | 322 g | 314 g | 282 g | 232 g | 192 g | 142 g |
| Oil phase | Extract of Haemato-coccus alga | 40 g | 40 g | 40 g | 40 g | 40 g | 40 g |
| | Mix tocopherol | 10 g | 10 g | 50 g | 100 g | 50 g | 100 g |
| | Soy bean oil | - | - | - | - | - | - |
| | Lecithin | 90 g | 90 g | 90 g | 90 g | 180 g | 180 g |
| | L-Ascorbyl palmitate | - | 8 g | - | - | - | - |
| Total | | 1000 g | 1000 g | 1000 g | 1000 g | 1000 g | 1000 g |

| | | E-07 Comparative Example | E-08 Comparative Example | E-09 Comparative Example | E-10 Comparative Example |
|---|---|---|---|---|---|
| Aqueous phase | Sucrose oleate | 13 g | 13 g | 13 g | - |
| | Decaglyceryl monooleate | 25 g | 25 g | 25 g | - |
| | Glycerin | 500 g | 500 g | 500 g | 500 g |
| | Pure water | 332 g | 322 g | 412 g | 360 g |
| Oil phase | Extract of Haemato-coccus alga | 40 g | 40 g | 40 g | 40 g |
| | Mix tocopherol | - | - | 10 g | 10 g |
| | Soy bean oil | - | 10 g | - | - |
| | Lecithin | 90 g | 90 g | - | 90 g |
| | L-Ascorbyl palmitate | - | - | - | - |
| Total | | 1000 g | 1000 g | 1000 g | 1000 g |

In the above table, sucrose oleate used is Ryoto Sugar ester O-1670 (HLB=15) manufactured by Mitsubishi Chemical Foods Corporation, and decaglyceryl monooleate used is NIKKOL Decaglyn 1-O (HLB=12) manufactured by Nikko Chemicals Co., Ltd. The extract of Haematococcus alga used is ASTOTS-S manufactured by Takedashiki Co., Ltd. Mix tocopherol used is Riken E Oil 800 manufactured by Riken Vitamin Co., Ltd. Lecithin (from soy beam) used is Lecion P manufactured by Riken Vitamin Co., Ltd. Also, soy bean oil and L-ascorbyl palmitate used are reagents manufactured by Wako Pure Chemical Industries, Ltd.

(Dilution)

1.0 g of each of the thus-obtained astaxanthin-containing emulsion compositions (E-01 to 10) is added to 99. 0 g of pure water, followed by stirring for 5 minutes at a rotation number of 10000 rpm in a homogenizer. The thus-obtained water-diluted emulsions are evaluated in the following manner. Results are shown in the following Table 2.

(Measurement of particle size)

Particle size of each of the water-diluted emulsions is measured by using a dynamic light-scattering particle sizeanalyzer, LB-550 (manufactured by Horiba, Ltd.

(Evaluation of stability of astaxanthin against decomposition)

Absorbance (Ci) of each of the water-diluted emulsions is measured by using ND-1000 Spectrophotometer manufactured by NanoDrop Technologies, Inc. Each of the water-diluted emulsions is placed in a lidded glass bottle and stored for 1 week in a thermostatic chamber kept at 50°C. After storing 1 week, absorbance is measured to determine absorbance (Cf) after storage. The astaxanthin-remaining ratio [%] is determined by calculating Cf/Ci x 100 for evaluation.

Table 2

| | E-01 Present Invention | E-02 Present Invention | E-03 Present Invention | E-04 Present Invention | E-05 Present Invention | E-06 Present Invention | E-07 Comparative Example | E-08 Comparative Example | E-09 Comparative Example | E-10 Comparative Example |
|---|---|---|---|---|---|---|---|---|---|---|
| Particle size [nm] | 50 | 55 | 67 | 85 | 60 | 75 | 52 | 52 | 51 | 285 |
| Astaxanthin-remaining ratio [%] | 69 | 74 | 81 | 84 | 83 | 87 | 3 | 5 | 23 | 70 |

It is seen from the results of Examples that the astaxanthin-containing emulsion compositions of the invention can have emulsified particles of a reduced particle size and have excellent stability against decomposition of astaxantins.

[Reference Examples]

Astaxanthin-containing compositions E-11 to 16 are obtained in absolutely the same manner as in Examples except for employing the formulations shown in the following Table 3.

Table 3

| | | E-11 Reference Example | E-12 Reference Example | E-13 Reference Example | E-14 Reference Example | E-15 Reference Example | E-16 Reference Example |
|---|---|---|---|---|---|---|---|
| Aqueous phase | Sucrose oleate | 13 g | 13 g | 13 g | 13 g | 13 g | 13 g |
| | Decaglyceryl monooleate | 25 g | 25 g | 25 g | 25 g | 25 g | 25 g |
| | Glycerin | - | 5 g | 10 g | 100 g | 600 g | 800 g |
| | Pure water | 822 g | 817 g | 812 g | 722 g | 222 g | 122 g |
| Oil phase | Extract of Haemato-coccus alga | 40 g | 40 g | 40 g | 40 g | 40 g | 40 g |
| | Mix tocopherol | 10 g | 10 g | 10 g | 10 g | 10 g | 10 g |
| | Lecithin | 90 g | 90 g | 90 g | 90 g | 90 g | 90 g |
| Total | | 1000 g | 1000 g | 1000 g | 1000 g | 1000 g | 1000 g |

(Emulsion stability)

Each of the emulsions is placed in a lidded glass bottle and is stored for 1 week in a thermostatic chamber kept at 60°C. After storing for 1 week, the emulsions are visually evaluated according to the following evaluation standard. Results are shown in Table 4 below together with the results of measurement of particle size.

A: No separation is observed between the oil phase and the aqueous phase.
B: Slight separation is observed between the oil phase and the aqueous phase.
C: Distinct separation is observed between the oil phase and the aqueous phase.

(Measurement of particle size)

Particle size is measured by diluting each of the emulsions in the same manner as in Examples.

Table 4

| | E-11 Reference Example | E-12 Reference Example | E-13 Reference Example | E-14 Reference Example | E-15 Reference Example | E-16 Reference Example |
|---|---|---|---|---|---|---|
| Emulsion stability | B | B | A | A | A | A |
| Particle size [nm] | 128 | 103 | 58 | 55 | 54 | 61 |

It is seen from the above results that storage stability of the emulsions can be enhanced and particle size can be reduced by incorporating glycerin in a content of from 10 to 60% by mass based on the mass of the compositions.
The carotenoid-containing emulsion composition of the invention contains emulsified particles having a small particle size and has excellent storage stability.
The entire disclosure of each and every foreign patent application from which the benefit of foreign priority has been claimed in the present application is incorporated herein by reference, as if fully set forth.

## Claims

**1.** A carotenoid-containing emulsion composition, which comprises carotenoid,
wherein the composition further comprises:

at least one water-soluble emulsifier in an aqueous phase; and
tocopherol and lecithin in an oil phase.

**2.** The carotenoid-containing emulsion composition according to claim 1,
wherein the carotenoid is at least one of astaxanthin and an ester thereof.

**3.** The carotenoid-containing emulsion composition according to claim 1,
wherein the at least one water-soluble emulsifier has an HLB of 10 or more.

**4.** The carotenoid-containing emulsion composition according to claim 1,
wherein the at least one water-soluble emulsifier is selected from the group consisting of sucrose fatty acid esters, polyglycerin fatty acid esters and sorbitan fatty acid esters.

**5.** The carotenoid-containing emulsion composition according to claim 1,
wherein a content of the carotenoid is from 0.1 to 10% by mass based on a mass of the composition,
a content of the lecithin is from 0.1 to 10% by mass based on a mass of the composition, and
a content of the tocopherol is from 0.1 to 5% by mass based on a mass of the carotenoid.

**6.** The carotenoid-containing emulsion composition according to claim 1, which further comprises glycerin.

7. The carotenoid-containing emulsion composition according to claim 6, wherein a content of the glycerin is from 10 to 60% by mass based on a mass of the composition.

8. The carotenoid-containing emulsion composition according to claim 1, which further comprises an antioxidant.

9. The carotenoid-containing emulsion composition according to claim 8, wherein the antioxidant is a radical scavenger.

10. The carotenoid-containing emulsion composition according to claim 1, wherein a particle size of emulsified particles is 200 nm or less.

11. A process for producing the carotenoid-containing emulsion composition according to claim 1, which comprises the following steps of:

a) dissolving the at least one water-soluble emulsifier in an aqueous medium to obtain an aqueous phase;
b) mixing and dissolving the carotenoid, the tocopherol and the lecithin, and optionally other fats and oils to obtain an oil phase; and
c) mixing the aqueous phase with the oil phase under stirring to obtain the emulsion composition.

12. The process according to claim 11, which further comprises:

performing high-pressure emulsification to reduce a particle size of emulsified particles of the emulsion composition.

13. A food, which comprises the carotenoid-containing emulsion composition according to claim 1.

14. A cosmetic product, which comprises the carotenoid-containing emulsion composition according to claim 1.

European Patent Office

# EUROPEAN SEARCH REPORT

Application Number
EP 07 01 1326

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/009160 A1 (VILLAMAR DANIEL F [US] ET AL) 15 January 2004 (2004-01-15)<br>* examples 1,2 * | 1-14 | INV.<br>A23D7/00<br>A23D7/01<br>A23K1/16<br>A23L1/275<br>A23L1/30<br>A61K9/107<br>A61Q1/06 |
| X | DATABASE WPI Week 200636<br>Derwent Publications Ltd., London, GB; AN 2006-348861<br>XP002448831<br>& JP 2006 129841 A (NIPPON OILS & FATS CO LTD) 25 May 2006 (2006-05-25)<br>* abstract * | 1-14 | |
| X | US 5 972 642 A (FLENOE BENT [DK] ET AL)<br>26 October 1999 (1999-10-26)<br>* example 11 * | 1-10,13,14 | |
| X | US 5 248 509 A (BRUIN SOLKE [NL])<br>28 September 1993 (1993-09-28)<br>* examples 10-12 * | 1-14 | |
| X | US 4 252 793 A (ALTMAN REINOUT F A)<br>24 February 1981 (1981-02-24)<br>* example 6 * | 1-14 | TECHNICAL FIELDS SEARCHED (IPC) |
| X | US 4 844 934 A (LUEDDECKE ERIK [DE] ET AL)<br>4 July 1989 (1989-07-04)<br>* claims 1-9; examples 1,2 * | 1-14 | A23D<br>A23K<br>A23L<br>A61K<br>A61Q |
| X | US 2 562 840 A (LEE CALDWELL ARCHIE)<br>31 July 1951 (1951-07-31)<br>* example 1 * | 1-14 | |
| X | WO 95/05747 A (PILLSBURY CO [US])<br>2 March 1995 (1995-03-02)<br>* page 6, paragraph 3 - page 7, paragraph 1; example 1 *<br>-/-- | 1-10 | |

The present search report has been drawn up for all claims

| Place of search | Date of completion of the search | Examiner |
|---|---|---|
| The Hague | 31 August 2007 | Rooney, Kevin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

3

EPO FORM 1503 03.82 (P04C01)

European Patent Office

# EUROPEAN SEARCH REPORT

Application Number
EP 07 01 1326

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2004/041247 A (BIODELIVERY SCIENCES INTERNATI [US]; MANNINO RAPHAEL J [US]; KRAUSE SA) 21 May 2004 (2004-05-21) * examples 3-7 * ----- | 1-14 | |
| X | WO 2004/000274 A (RADICS ENDRE D [HU]) 31 December 2003 (2003-12-31) * example 1 * ----- | 1-14 | |
| X | EP 0 870 435 A1 (TAIYO KAGAKU KK [JP]) 14 October 1998 (1998-10-14) * page 13, paragraph 1 * ----- | 1-14 | |
| X | EP 1 264 595 A (PACIFIC CORP [KR]) 11 December 2002 (2002-12-11) * paragraph [0033] * ----- | 1-14 | |
| X | US 6 265 180 B1 (ZUELLI FRED [CH] ET AL) 24 July 2001 (2001-07-24) * example 11 * ----- | 1-14 | TECHNICAL FIELDS SEARCHED (IPC) |
| X | WO 99/56563 A (ABBOTT LAB [US]) 11 November 1999 (1999-11-11) * example 4 * ----- | 1-14 | |
| X | US 2005/089516 A1 (AMANO SATOSHI [JP] ET AL) 28 April 2005 (2005-04-28) * examples 5,6 * ----- | 1-14 | |
| A | WO 2005/065652 A1 (FRIEDMAN DORON [IL]) 21 July 2005 (2005-07-21) * the whole document * ----- | 1-14 | |

The present search report has been drawn up for all claims

3

| Place of search | Date of completion of the search | Examiner |
|---|---|---|
| The Hague | 31 August 2007 | Rooney, Kevin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 07 01 1326

This annex lists the patent family members relating to the patent documents cited in the above-mentioned European search report.
The members are as contained in the European Patent Office EDP file on
The European Patent Office is in no way liable for these particulars which are merely given for the purpose of information.

31-08-2007

| Patent document cited in search report | | Publication date | Patent family member(s) | | | Publication date |
|---|---|---|---|---|---|---|
| US 2004009160 | A1 | 15-01-2004 | NONE | | | |
| JP 2006129841 | A | 25-05-2006 | NONE | | | |
| US 5972642 | A | 26-10-1999 | NONE | | | |
| US 5248509 | A | 28-09-1993 | NONE | | | |
| US 4252793 | A | 24-02-1981 | NONE | | | |
| US 4844934 | A | 04-07-1989 | AU | 593081 | B2 | 01-02-1990 |
| | | | AU | 7062887 | A | 01-10-1987 |
| | | | DE | 3610191 | A1 | 01-10-1987 |
| | | | EP | 0239086 | A2 | 30-09-1987 |
| | | | IL | 81973 | A | 23-12-1990 |
| | | | JP | 7096649 | B | 18-10-1995 |
| | | | JP | 62240364 | A | 21-10-1987 |
| US 2562840 | A | 31-07-1951 | NONE | | | |
| WO 9505747 | A | 02-03-1995 | AU | 7633194 | A | 21-03-1995 |
| WO 2004041247 | A | 21-05-2004 | AU | 2003296923 | A1 | 07-06-2004 |
| | | | CA | 2504329 | A1 | 21-05-2004 |
| | | | EP | 1562557 | A2 | 17-08-2005 |
| | | | JP | 2006514103 | T | 27-04-2006 |
| WO 2004000274 | A | 31-12-2003 | AU | 2003244885 | A1 | 06-01-2004 |
| | | | HU | 0202032 | A2 | 28-10-2004 |
| EP 0870435 | A1 | 14-10-1998 | AU | 4398897 | A | 24-04-1998 |
| | | | CA | 2239035 | A1 | 09-04-1998 |
| | | | DE | 69727208 | D1 | 19-02-2004 |
| | | | DE | 69727208 | T2 | 25-11-2004 |
| | | | DK | 870435 | T3 | 24-05-2004 |
| | | | ES | 2214638 | T3 | 16-09-2004 |
| | | | WO | 9814072 | A1 | 09-04-1998 |
| | | | US | 6074675 | A | 13-06-2000 |
| EP 1264595 | A | 11-12-2002 | JP | 2003026604 | A | 29-01-2003 |
| | | | KR | 20020092622 | A | 12-12-2002 |
| | | | US | 2003078238 | A1 | 24-04-2003 |
| US 6265180 | B1 | 24-07-2001 | CA | 2264441 | A1 | 30-09-1999 |
| | | | EP | 0953632 | A1 | 03-11-1999 |
| | | | JP | 11318436 | A | 24-11-1999 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 07 01 1326

This annex lists the patent family members relating to the patent documents cited in the above-mentioned European search report.
The members are as contained in the European Patent Office EDP file on
The European Patent Office is in no way liable for these particulars which are merely given for the purpose of information.

31-08-2007

| Patent document cited in search report | | Publication date | Patent family member(s) | | | Publication date |
|---|---|---|---|---|---|---|
| WO 9956563 | A | 11-11-1999 | AU | 746236 | B2 | 18-04-2002 |
| | | | AU | 3780599 | A | 23-11-1999 |
| | | | BR | 9910279 | A | 02-10-2001 |
| | | | CA | 2330061 | A1 | 11-11-1999 |
| | | | EP | 1102549 | A2 | 30-05-2001 |
| | | | US | 6475539 | B1 | 05-11-2002 |
| US 2005089516 | A1 | 28-04-2005 | NONE | | | |
| WO 2005065652 | A1 | 21-07-2005 | CA | 2547712 | A1 | 21-07-2005 |
| | | | EP | 1706100 | A1 | 04-10-2006 |
| | | | JP | 2007517859 | T | 05-07-2007 |
| | | | US | 2007190080 | A1 | 16-08-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2049091 A
- JP 5155736 A
- JP 9328419 A
- JP 2005506841 T
- JP 8103288 A
- JP 5068585 A


### Non-patent literature cited in the description


- Nippon Koshohin Kagakukai. 1994, 66
- **DAVIES, B.H.** Chemistry and Biochemistry of Plant Pigments. Academic Press, 1976, 38-165
- **KUHN, R. ; SOERENSEN, N.A.** The coloring matters of the lobster (Astacus gammarus L. *Angew. Chem.,* 1938, vol. 51, 465-466
- **CHEESMAN, D.F.** Ovorubin, a chromoprotein from the eggs of the gastropod mollusc Pomacea canaliculata. *Proc. Roy. Soc. B,* 1958, vol. 149, 571-587
- **YAMAGUCHI, K ; MIKI, W ; TORIU, N ; KONDO, Y ; MURAKAMI, M ; KONOSU, S ; SATAKE, M ; FUJITA, T.** The composition of carotenoid pigments in the Antarctic krill Euphausia superba. *Bull. Jap. Soc. Sci. Fish,* 1983, vol. 49, 1411-1415
- **RENSTROM, B ; LIAAEN-JENSEN, S.** Fatty acids of some esterified carotenols. *Comp. Biochem. Physiol. B., Comp. Biochem.,* 1981, vol. 69, 625-627
- **ANDREWES, A.G. ; STARR, M.P.** 3R,3'R)-Asttaxanthin from the yeast Phaffa rhodozyma. *Phytochem.,* 1976, vol. 15, 1009-1011
- **ANDREWES, A.G. ; PHAFFIA, H.J. ; STARR, M. P.** Carotenids of Phaffa rhodozyma, a red pigmented fermenting yeast. *Phytochem.,* 1976, vol. 15, 1003-1007
- *Astaxanthin no Kagaku (Chemistry of Astaxanthin,* 2005, http://www.astaxanthin.co.jp/chemical/basic.htm
- Yukagaku Binran. 2001
- Kosankazai no Riron to Jissai (Theory and Practice of Antioxidants. San Shobo, 1984
- Sanka Bosizai Handobukku (Handbook of Antioxidants. Taiseisha, 1976